# EUROPEAN PATENT APPLICATION

(11) **EP 4 382 200 A1**
(43) Date of publication of application: **12.06.2024**
(21) Application number: 22852821.2
(22) Date of filing: 19.07.2022
(51) Int. Cl.: B01J 20/22, B01D 53/14, B01D 53/62, B01D 53/81, B01D 53/96, B01J 20/28, B01J 20/34, C01B 32/50, C07C 211/18

(54) **CARBON DIOXIDE ABSORBENT, METHOD FOR RECOVERING CARBON DIOXIDE, AND APPARATUS FOR SEPARATING AND RECOVERING CARBON DIOXIDE**

(30) Priority: 05.08.2021 JP 2021129200; 28.03.2022 JP 2022052196
(71) Applicant: MITSUBISHI GAS CHEMICAL COMPANY, INC., Chiyoda-ku Tokyo 100-8324 (JP)
(72) Inventor: KOUNO, Kazuki, Hiratsuka-shi, Kanagawa 254-0016 (JP); MAKINOSHIMA, Takashi, Hiratsuka-shi, Kanagawa 254-0016 (JP); KAWASHIMA, Yuki, Hiratsuka-shi, Kanagawa 254-0016 (JP)
(74) Representative: Hoffmann Eitle
(86) International application number: PCT/JP2022/028021
(87) International publication number: WO 2023/013397

(57) **Abstract**

A carbon dioxide absorbent containing a cyclic amine compound (A) and a porous material (B), wherein the cyclic amine compound (A) has 35% by mol or more of a primary amino group with respect to total amino groups; a method for capturing carbon dioxide using the carbon dioxide absorbent; and a carbon dioxide separation and capture apparatus.

## Description

### Technical Field

The present invention relates to a carbon dioxide absorbent, a method for capturing carbon dioxide, and a carbon dioxide separation and capture apparatus.

### Background Art

From a viewpoint of global warming issues, there is a need to reduce carbon dioxide.

One of methods for reducing carbon dioxide is a method for capturing carbon dioxide using the carbon dioxide absorbent. As the carbon dioxide absorbent, aqueous solutions of amine compounds such as monoethanolamine are generally used. The aqueous solutions of the amine compounds have a property that they do not release carbon dioxide absorbed if not a high temperature of, e.g., 120°C or more, and if a carbon dioxide release temperature is set above the boiling point of water, a lot of energy is required to capture carbon dioxide because of high latent and specific heat of water.

As described above, one issue with respect to conventional carbon dioxide absorbents is further energy conservation when separating and capturing carbon dioxide. Another issue with respect to the conventional carbon dioxide absorbents is to reduce volatility of the amine compounds contained in the carbon dioxide absorbents, because a small amount of the amine compounds is volatilized and lost when gas is brought into contact with them in a step of absorbing carbon dioxide.

In addition, in a conventional chemical absorption method, the carbon dioxide absorbents are regenerated by boiling them with steam heating until a temperature of around 110°C to 130°C. Therefore, this method requires a very large amount of thermal energy. Furthermore, the thermal stability of the carbon dioxide absorbent is also an issue because there is concern that the amine compounds contained in the carbon dioxide absorbents may thermally decompose during this regeneration step.

Recently, the carbon dioxide absorbent having the amine compound supported on a porous material has been studied. Solidifying the amine compound by supporting it on the porous material enables carbon dioxide to be captured at lower energy than those of the aqueous solutions, of which the high latent and specific heats are problems.

Technologies related to such solidified carbon dioxide absorbents includes, for example, those described in Patent Literatures 1 to 3.

Patent Literature 1 discloses a solid absorbent for separating and capturing carbon dioxide which contains a specific alkanolamine and in which the alkanolamine is supported on a support.

Patent Literature 2 discloses a carbon dioxide absorbent, in which an amine compound is supported onto porous particles in which hydrophilic fibers and porous powder are composited by a hydrophilic binder.

Patent Literature 3 discloses a carbon dioxide absorbent composition containing polyethylene polyamine, phosphoric acid and/or phosphate, and silica.

### Citation List

### Patent Literature

PTL1: JP 2012-139622 A
PTL2: JP 2018-187574 A
PTL3: JP 2020-58966 A

### Summary of Invention

### Technical Problem

According to studies of the present inventors, it has become apparent that carbon dioxide absorbents containing an amine compound and a porous material, such as those described in Patent Literatures 1 to 3, have room for improvement in terms of repeated usability.

The present invention has been made in view of the above circumstances, and provides a carbon dioxide absorbent with improved repeated usability, a method for capturing carbon dioxide using the carbon dioxide absorbent, and a carbon dioxide separation and capture apparatus.

### Solution to Problem

The present inventors have made intensive studies in order to solve the above problem. As a result, they have found that a carbon dioxide absorbent containing a specific cyclic amine compound (A) and a porous material can improve the repeated usability, and have completed the present invention.

That is, according to the present invention, provided are a carbon dioxide absorbent, a method for capturing carbon dioxide, and a carbon dioxide separation and capture apparatus as follows.
[1] A carbon dioxide absorbent comprising a cyclic amine compound (A) and a porous material (B), wherein the cyclic amine compound (A) has 35% by mol or more of a primary amino group with respect to total amino groups.
[2] The carbon dioxide absorbent according to the above [1], wherein at least some of the cyclic amine compound (A) is supported on the porous material (B).
[3] The carbon dioxide absorbent according to the above [1] or [2], wherein the porous material (B) comprises at least one selected from the group consisting of silica and alumina.
[4] The carbon dioxide absorbent according to any one of the above [1] to [3], wherein the porous material (B) is in a particulate form.
[5] The carbon dioxide absorbent according to the above [4], wherein a volume median particle size (D₅₀) of the porous material (B) as measured by laser diffraction/scattering particle size distribution measurement is 1 µm or more and 500 µm or less.
[6] The carbon dioxide absorbent according to any one of the above [1] to [5], wherein a specific surface area of the porous material (B) as measured by a BET method is 2 m²/g or more and 3,000 m²/g or less.
[7] The carbon dioxide absorbent according to any one of the above [1] to [6], wherein a pore volume of the porous material (B) is 0.1 cm³/g or more and 5.0 cm³/g or less.
[8] The carbon dioxide absorbent according to any one of the above [1] to [7], wherein a content of the cyclic amine compound (A) is 0.1 parts by mass or more and 1,000 parts by mass or less with respect to 100 parts by mass of the porous material (B).
[9] The carbon dioxide absorbent according to any one of the above [1] to [8], wherein the cyclic amine compound (A) comprises at least one selected from the group consisting of an amine compound (a1) represented by the following formula (1) and an amine compound (a2) having a heterocyclic structure selected from the group consisting of an oxygen-containing heterocyclic structure and a sulfur-containing heterocyclic structure, 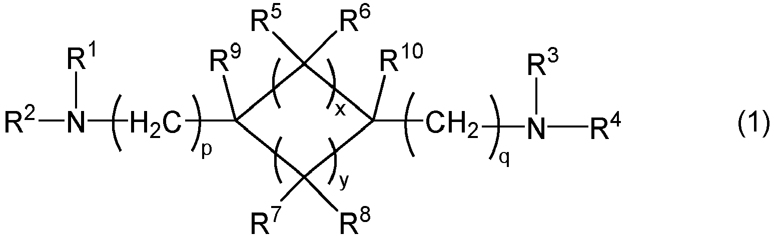 wherein R¹ to R⁴ each independently represent a hydrogen atom, or a hydrocarbon group having 1 or more and 10 or less carbon atoms and optionally having at least one substituent selected from the group consisting of an amino group, a cyano group and a phenyl group; R⁵ to R¹⁰ each independently represent a hydrogen atom or a hydrocarbon group having 1 or more and 4 or less carbon atoms; x and y each independently represent an integer of 0 or more and 6 or less; x + y is 1 or more and 6 or less; p and q each independently represent an integer of 0 or more and 4 or less; and at least one of p and q is 1 or more.
[10] The carbon dioxide absorbent according to any one of the above [1] to [9], wherein a maximum carbon dioxide release temperature of the cyclic amine compound (A) is 140°C or less measured by the following method:
   heating the cyclic amine compound (A) with carbon dioxide absorbed from 23°C to 250°C at 10°C/minute of a heating rate, measuring a temperature at which an endothermic amount associated with desorption of carbon dioxide reaches a maximum, and letting the temperature be the maximum carbon dioxide release temperature.
[11] The carbon dioxide absorbent according to any one of the above [1] to [10], wherein a molecular weight of the cyclic amine compound (A) is 90 or more and 1,000 or less.
[12] The carbon dioxide absorbent according to any one of the above [1] to [11], wherein an amine value of the cyclic amine compound (A) is 400 mgKOH/g or more and 1,500 mgKOH/g or less.
[13] The carbon dioxide absorbent according to any one of the above [1] to [12], wherein the number of amino groups in the cyclic amine compound (A) is 1 or more and 6 or less.
[14] The carbon dioxide absorbent according to any one of the above [1] to [13], wherein a cyclic structure of the cyclic amine compound (A) comprises at least one selected from the group consisting of a 5-membered ring and a 6-membered ring.
[15] The carbon dioxide absorbent according to any one of the above [1] to [14], wherein the cyclic amine compound (A) comprises at least one selected from the group consisting of bis(aminomethyl)cyclohexane and derivatives thereof, limonenediamine and derivatives thereof, isophoronediamine and derivatives thereof, 2,5-bisaminomethylfuran and derivatives thereof, 2,5-bis(aminomethyl)tetrahydrofuran and derivatives thereof, furfurylamine and derivatives thereof, tetrahydrofurfurylamine and derivatives thereof, 4-aminomethyltetrahydropyran and derivatives thereof, 4-(2-aminoethyl)morpholine and derivatives thereof, and 2-thiophenemethylamine and derivatives thereof.
[16] A method for capturing carbon dioxide, comprising using the carbon dioxide absorbent according to any one of the above [1] to [15],
[17] The method according to the above [16], wherein the method comprises:
   an absorption step of bringing a gas containing carbon dioxide into contact with the carbon dioxide absorbent, to cause the carbon dioxide absorbent to absorb the carbon dioxide, and
   a desorption step of desorbing carbon dioxide from the carbon dioxide absorbent with carbon dioxide absorbed in the absorption step, and
   wherein the desorption step comprises at least one step selected from the group consisting of the following (I) to (III):
      (I) a step of subjecting the carbon dioxide absorbent with carbon dioxide absorbed to a reduced pressure condition;
      (II) a step of bringing an inert gas not containing carbon dioxide into contact with the carbon dioxide absorbent with carbon dioxide absorbed; and
      (III) a step of heating the carbon dioxide absorbent with carbon dioxide absorbed.
[18] The method according to the above [17], wherein a temperature at which the gas containing carbon dioxide is brought into contact with the carbon dioxide absorbent in the absorption step is 0°C or more and less than 60°C.
[19] The method according to the above [17] or [18], wherein a heating temperature in the step (III) is 50°C or more and 120°C or less.
[20] A carbon dioxide separation and capture apparatus comprising:
   an absorption apparatus comprising a mechanism for bringing a gas containing carbon dioxide into contact with the carbon dioxide absorbent according to any one of the above [1] to [15], to cause the carbon dioxide absorbent to absorb the carbon dioxide; and
   a desorption apparatus comprising a mechanism for desorbing carbon dioxide from the carbon dioxide absorbent with carbon dioxide absorbed.

### Advantageous Effects of Invention

According to the present invention, a carbon dioxide absorbent having improved repeated usability, a method for capturing carbon dioxide using the carbon dioxide absorbent, and a carbon dioxide separation and capture apparatus can be provided.

### Brief Description of Drawing

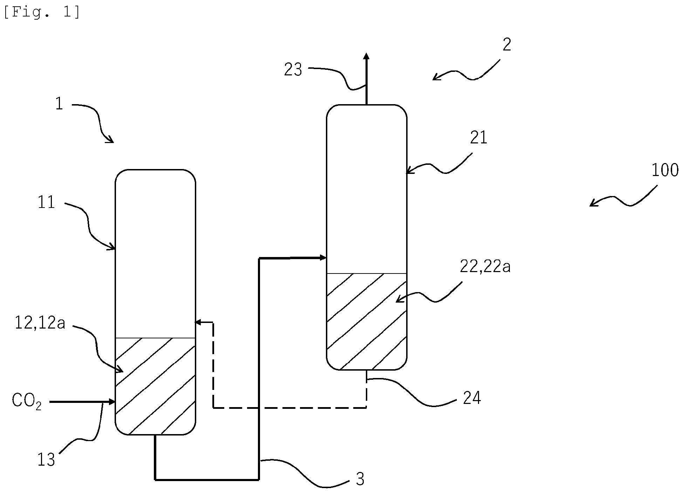

[Fig. 1] Fig. 1 is a schematic diagram showing an embodiment of a carbon dioxide separation and capture apparatus of the present invention.

### Description of Embodiments

Embodiments of the present invention (hereinafter, simply referred to as "the present embodiments") will be described in detail. The following embodiments are examples to explain the present invention and do not limit the contents of the present invention. The present invention can be implemented with appropriate modifications within the scope of the invention. In the present embodiments, it is considered that provisions that are preferred can be adopted arbitrarily, and combinations of preferred ones are more preferred. In the present embodiments, description of "XX to YY" means "XX or more and YY or less".

### [Carbon Dioxide Absorbent]

A carbon dioxide absorbent of the present invention comprises a cyclic amine compound (A) and a porous material (B), wherein the cyclic amine compound (A) has 35% by mol or more of a primary amino group with respect to total amino groups.

The carbon dioxide absorbent of the present invention comprises the cyclic amine compound (A) and the porous material (B), and is an absorbent with improved repeated usability. Also, the carbon dioxide absorbent of the present invention has good absorption performance for carbon oxide from the air.

In the present embodiment, "repeated usability" means a retention rate of an amount of carbon dioxide absorbed when cycle tests of absorption and desorption of carbon dioxide are performed. Also, in the present embodiment, "good in the amount of carbon dioxide absorbed from the air" means a large in the amount absorbed for carbon dioxide at a low concentration (about 0.04% by volume) from the air. "Primary amino group" means an amino group having two hydrogen atoms on a nitrogen atom, i.e., -NH₂ group.

The carbon dioxide absorbent of the present invention includes the cyclic amine compound (A) and the porous material (B). Including the cyclic amine compound (A) can provide improvements of the repeated usability and the amount of carbon dioxide absorbed from the air. The reason thereof is not certain, but is considered to be as follows.

The cyclic amine compound (A) has a cyclic structure that is resistant to an oxidation reaction, and therefore, the oxidation reaction and weight loss are unlikely to occur even if the compound is heat-treated when desorbing carbon dioxide. Furthermore, since the cyclic amine compound (A) has a cyclic structure that does not easily absorb water, the amount of energy required to volatilize water, which is necessary when desorbing carbon dioxide, is low, and carbon dioxide is easily desorbed. For these reasons, the carbon dioxide absorbent of the present invention is expected to improve the repeated usability. Still, an acyclic aliphatic amine compound is considered to be inferior in the repeated usability because it is prone to generate cyclization reaction, oxidation reaction, weight loss, etc. upon heating treatment.

In addition, the cyclic amine compound (A) has 35% by mol or more of the primary amino group with respect to the total amino groups. Such an amino group is considered to have low steric hindrance and to be easy to absorb carbon dioxide. Therefore, the carbon dioxide absorbent of the present invention is considered to be able to improve the amount of carbon dioxide absorbed from the air.

In the carbon dioxide absorbent according to the present invention, from a viewpoint that carbon dioxide can be captured with lower energy, it is preferable that at least some of the cyclic amine compound (A) is supported on the porous material (B), and more preferable that the cyclic amine compound (A) is supported and solidified on the porous material (B).

The carbon dioxide absorbent according to the present invention can be suitably used in technology of directly absorbing carbon dioxide (DAC) from the air because of its good absorption performance for carbon oxide from the air.

The carbon dioxide absorbent according to the present invention can be also suitably used in case of capturing carbon dioxide at low concentrations of, e.g., 0.01% by volume or more and 1% by volume or less.

### <Cyclic Amine Compound (A)>

The cyclic amine compound (A) has 35% by mol or more of the primary amino group with respect to the total amino groups contained in the cyclic amine compound (A), and from a viewpoint of further improving the repeated usability and the amount of carbon dioxide absorbed from the air, preferably 40% by mol or more of the primary amino group, more preferably 45% by mol or more, and even more preferably 50% by mol or more, and preferably 100% by mol or less, more preferably 75% by mol or less, and even more preferably 70% by mol or less.

The cyclic amine compound (A) is an amine compound having a cyclic structure.

The cyclic structure of the cyclic amine compound (A) includes, for example, an alicyclic hydrocarbon structure, an aromatic hydrocarbon structure, a heterocyclic structure containing a heteroatom in a ring, and from the viewpoint of further improving the repeated usability and the amount of carbon dioxide absorbed from the air, preferably comprises at least one selected from the group consisting of the alicyclic hydrocarbon structure and the heterocyclic structure, more preferably comprises at least one selected from the group consisting of the alicyclic hydrocarbon structure, an oxygen-containing heterocyclic structure, and a sulfur-containing heterocyclic structure, and even more preferably comprises the alicyclic hydrocarbon structure.

Here, in the present embodiment, the alicyclic hydrocarbon structure refers to a cyclic structure composed of saturated or unsaturated carbon and hydrogen without aromaticity; the heterocyclic structure refers to a heterocyclic structure containing the heteroatom in a ring; and the oxygen-containing heterocyclic structure or the sulfur-containing heterocyclic structure refers to a heterocyclic structure containing an oxygen atom or a sulfur atom as the heteroatom in a ring structure.

The oxygen-containing heterocyclic structure according to the present embodiment is preferably a heterocyclic structure containing a nitrogen atom and an oxygen atom as the heteroatoms in the ring structure, or a heterocyclic structure containing only oxygen atoms as the heteroatoms in the ring structure, more preferably a heterocyclic structure containing only one oxygen atom as the heteroatom in the ring structure, or a heterocyclic structure containing only one oxygen atom and only one nitrogen atom as the heteroatoms in the ring structure, and even more preferably a heterocyclic structure containing only one oxygen atom as the heteroatom in the ring structure.

In addition, the sulfur-containing heterocyclic structure according to the present embodiment is preferably a heterocyclic structure containing a nitrogen atom and a sulfur atom as the heteroatoms in the ring structure, or a heterocyclic structure containing only sulfur atoms as the heteroatoms in the ring structure, more preferably a heterocyclic structure containing only one sulfur atom as the heteroatom in the ring structure, or a heterocyclic structure containing only one sulfur atom and only one nitrogen atom as the heteroatoms in the ring structure, and even more preferably a heterocyclic structure containing only one sulfur atom as the heteroatom in the ring structure.

The cyclic amine compound (A) may be any of a cis-isomer, a trans-isomer, or a mixture of the cis-isomer and the trans-isomer, if it has a structure which can take both of the cis-isomer and the trans-isomer.

The cyclic structure of the cyclic amine compound (A) preferably comprises at least one selected from the group consisting of a 5-membered ring and a 6-membered ring, and more preferably the 6-membered ring, from the viewpoint of further improving the repeated usability and the amount of carbon dioxide absorbed from the air.

In addition, the cyclic amine compound (A) preferably has one cyclic structure from the viewpoint of further improving the repeated usability and the amount of carbon dioxide absorbed from the air. In other words, it is preferable that the cyclic amine compound (A) is a monocyclic compound.

The alicyclic hydrocarbon structure of the cyclic amine compound (A) includes, for example, a cyclopropane ring, a cyclobutane ring, a cyclopentane ring, a cyclohexane ring, a cycloheptane ring, a cyclooctane ring. Among the above ring structures, the cyclopentane ring and the cyclohexane ring are preferable, the cyclohexane ring is more preferable, and a 1,3-substituted cyclohexane ring is even more preferable.

Number of the amino groups in the cyclic amine compound (A) is preferably 1 or more, more preferably 2 or more, and preferably 6 or less, from the viewpoint of further improving the repeated usability and the amount of carbon dioxide absorbed from the air.

In addition, the amino group is preferably an amino group having a nitrogen-hydrogen bond, more preferably at least one amino group selected from the group consisting of the primary amino group and a secondary amino group, and even more preferably the primary amino group, from the viewpoint of further improving the amount of carbon dioxide absorbed from the air.

The cyclic amine compound (A) is preferably at least one selected from the group consisting of an amine compound (a1) represented in the following formula (1) and an amine compound (a2) having the heterocyclic structure selected from the group consisting of the oxygen-containing heterocyclic structure and the sulfur-containing heterocyclic structure, from the viewpoint of further improving the repeated usability and the amount of carbon dioxide absorbed from the air.

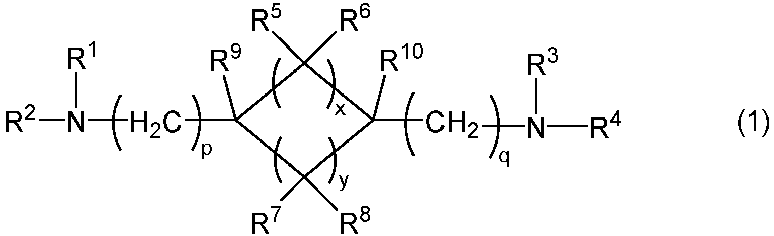

In the above formula (1), R¹ to R⁴ each independently represent a hydrogen atom, or a hydrocarbon group having 1 or more and 10 or less carbon atoms and optionally having at least one substituent selected from the group consisting of an amino group, a cyano group, and a phenyl group; R⁵ to R¹⁰ each independently represent a hydrogen atom or a hydrocarbon group having at least 1 or more and 4 or less carbon atoms; x and y each independently represent an integer of 0 or more and 6 or less; x + y is 1 or more and 6 or less; p and q each independently represent an integer of 0 or more and 4 or less; and at least one of p and q is 1 or more.

R¹ to R⁴ each independently represents the hydrogen atom, or the hydrocarbon group having 1 or more and 10 or less carbon atoms, and optionally having at least one substituent selected from the group consisting of the amino group, the cyano group and the phenyl group, preferably the hydrogen atom, or an alkyl group having 1 or more and 4 or less carbon atoms, and optionally having at least one substituent selected from the group consisting of the amino group, the cyano group and the phenyl group, more preferably the hydrogen atom, or the alkyl group having 1 or more and 4 or less carbon atoms, and optionally having at least one substituent selected from the group consisting of the amino group and the cyano group, even more preferably the hydrogen atom or the alkyl group having 2 or more and 4 or less carbon atoms, and optionally having at least one substituent selected from the group consisting of the amino group and the cyano group.

The number of carbon atoms of the hydrocarbon groups of R¹ to R⁴ is each independently 1 or more, preferably 2 or more, and 10 or less, preferably 5 or less, more preferably 4 or less, and even more preferably 3 or less.

R⁵ to R¹⁰ each independently represents the hydrogen atom or the hydrocarbon group having 1 or more and 4 or less carbon atoms, preferably the hydrogen atom or the alkyl group having 1 or more and 4 or less carbon atoms, more preferably the hydrogen atom or the alkyl group having 1 or more and 3 or less carbon atoms, even more preferably the hydrogen atom or a methyl group, and still more preferably the hydrogen atom.

The hydrocarbon groups of R⁵ to R¹⁰ have each independently 1 or more and 4 or less carbon atoms, preferably 1 or 2, and more preferably 1.

p and q each independently represents 0 or more, preferably 1 or more, and 4 or less, preferably 2 or less, more preferably 1. However, at least one of p and q is 1 or more.

x and y each independently represent an integer of 0 or more and 6 or less, and x + y is 1 or more and 6 or less. From viewpoints of increasing the steric hindrance of the whole molecule and further improving the repeated usability and the amount of carbon dioxide absorbed from the air, x + y is preferably 2 or more, more preferably 3 or more, and even more preferably 4 or more, and from the viewpoint of improving the amount of carbon dioxide absorbed, x + y is preferably 5 or less, and more preferably 4. In other words, the alicyclic hydrocarbon structure is preferably the 5-membered ring or the 6-membered ring, and more preferably the 6-membered ring. When x + y is 4, preferably x is 1 and y is 3.

As the cyclic amine compound (A), from the viewpoint of further improving the repeated usability and the amount of carbon dioxide absorbed from the air, preferable is at least one selected from the group consisting of o-xylylenediamine and derivatives thereof, m-xylylenediamine and derivatives thereof, p-xylylenediamine and derivatives thereof, bis(aminomethyl)cyclohexane and derivatives thereof, limonenediamine and derivatives thereof, isophoronediamine and derivatives thereof, 2,5-bisaminomethylfuran and derivatives thereof, 2,5-bis(aminomethyl)tetrahydrofuran and derivatives thereof, furfurylamine and derivatives thereof, tetrahydrofurfurylamine and derivatives thereof, 4-aminomethyltetrahydropyran and derivatives thereof, 4-(2-aminoethyl)morpholine and derivatives thereof, and 2-tiophenemethylamine and derivatives thereof; more preferable is at least one selected from the group consisting of bis(aminomethyl)cyclohexane and derivatives thereof, limonenediamine and derivatives thereof, isophoronediamine and derivatives thereof, 2,5-bisaminomethylfuran and derivatives thereof, 2,5-bis(aminomethyl)tetrahydrofuran and derivatives thereof, furfurylamine and derivatives thereof, tetrahydrofurfurylamine and derivatives thereof, 4-aminomethyltetrahydropyran and derivatives thereof, 4-(2-aminoethyl)morpholine and derivatives thereof, and 2-thiophenemethylamine and derivatives thereof; even more preferable is at least one selected from the group consisting of bis(aminomethyl)cyclohexane and derivatives thereof, limonenediamine and derivatives thereof, and isophoronediamine and derivatives thereof; still more preferable are bis(aminomethyl)cyclohexane and derivatives thereof; yet more preferable are 1,3-bis(aminomethyl)cyclohexane and derivatives thereof; even much more preferable are derivatives of 1,3-bis(aminomethyl)cyclohexane; still much more preferable is at least one selected from the group consisting of derivatives of 1,3-bis(aminomethyl)cyclohexane represented by the following formula (2), Formula (3), Formula (4), or Formula (5); yet much more preferable is at least one selected from the group consisting of derivatives of 1,3-bis(aminomethyl)cyclohexane represented by the following formula (3) or Formula (5); and even still much more preferable are derivatives of 1,3-bis(aminomethyl)cyclohexane represented by the following formula (5).

Here, the derivative of the above various amine includes, for example, a compound in which at least one of hydrogen atoms in the amino group is substituted by the hydrocarbon group having 1 or more and 10 or less carbon atoms, optionally having at least one substituent selected from the group consisting of the amino group, the cyano group and the phenyl group, preferably the alkyl group having 1 or more and 4 or less carbon atoms, and optionally having at least one substituent selected from the group consisting of the amino group, the cyano group and the phenyl group, more preferably the alkyl group having 1 or more and 4 or less carbon atoms, and optionally having at least one substituent selected from the group consisting of the amino group and the cyano group, even more preferably the alkyl group having 2 or more and 4 or less carbon atoms, and optionally having at least one substituent selected from the group consisting of the amino group and the cyano group.

In addition, derivatives of the above various amines include, for example, compounds in which at least some of hydrogen atoms in the cyclic structure are substituted with a hydrocarbon group having 1 or more and 4 or less carbon atoms, preferably with an alkyl group having 1 or more and 3 or less carbon atoms, more preferably with a methyl group or an ethyl group, and even more preferably with the methyl group.

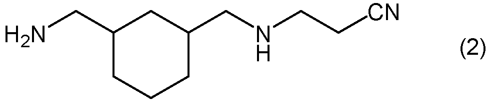

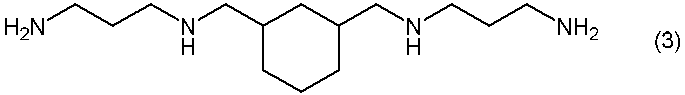

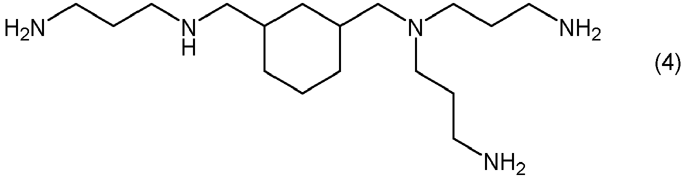

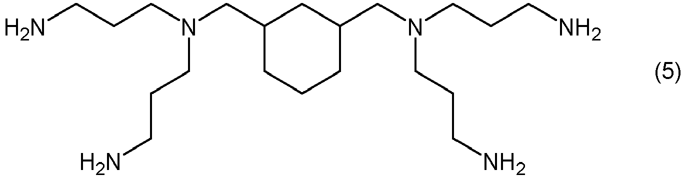

Theses cyclic amine compounds (A) can be used alone or in combination of two or more.

A content of the cyclic amine compound (A) in the carbon dioxide absorbent according to the present invention is, from the viewpoint of further improving the repeated usability and the amount of carbon dioxide absorbed from the air, preferably 0.1 parts by mass or more, more preferably 1 part by mass or more, even more preferably 10 parts by mass or more, still more preferably 25 parts by mass or more, and yet more preferably 50 parts by mass or more, and preferably 1,000 parts by mass or less, more preferably 500 parts by mass or less, even more preferably 250 parts by mass or less, still more preferably 200 parts by mass or less, and yet more preferably 150 parts by mass or less, with respect to 100 parts by mass of the porous material (B).

In addition, the content of the cyclic amine compound (A) in the carbon dioxide absorbent according to the present invention is, from the viewpoint of further improving the repeated usability and the amount of carbon dioxide absorbed from the air, preferably 50 parts by mass or more, more preferably 60 parts by mass or more, even more preferably 70 parts by mass or more, still more preferably 80 parts by mass or more, yet more preferably 90 parts by mass or more, even much more preferably 95 parts by mass or more, and preferably 100 parts by mass or less, when the total amount of the amine compound contained in the carbon dioxide absorbent is 100 parts by mass.

A maximum carbon dioxide release temperature of the cyclic amine compound (A) as measured by the following method is, from viewpoints of improving carbon dioxide desorption performance and further improving the repeated usability, preferably 140°C or less, more preferably 130°C or less, even more preferably 120°C or less, still more preferably 110°C or less, and yet more preferably 105°C or less. A lower limit value of the above maximum carbon dioxide release temperature is not limited, but is, e.g., 40°C or more.

### (Method)

The cyclic amine compound (A) is heated with carbon dioxide absorbed from 23°C to 250°C at 10°C/minute of a heating rate, and a temperature at which an endothermic amount associated with desorption of carbon dioxide reaches a maximum is measured, and this temperature is taken as the maximum carbon dioxide release temperature. Here, the cyclic amine compound (A) with carbon dioxide absorbed can be prepared, for example, by allowing 5 mmol of the cyclic amine compound (A) to stand in the air at 23°C and 50% RH for 24 hours.

An acid dissociation constant (pKa) of the cyclic amine compound (A) is preferably 8.0 or more, more preferably 9.0 or more, even more preferably 9.3 or more, from the viewpoint of further improving the amount of carbon dioxide absorbed from the air, and preferably 12.0 or less, and more preferably 11.0 or less, from the viewpoints of improving the carbon dioxide desorption performance and further improving the repeated usability.

In the present embodiment, the acid dissociation constant of the cyclic amine compound (A) is a value obtained by the following measurement method based on an acid-base titration method.
(1) Dissolving 0.2 g of the cyclic amine compound (A) in 30 mL of purified water.
(2) Calculating the acid dissociation constant (pKa) by titrating the solution obtained in the above (1) with 0.1 N perchloric acid-acetic acid solution using an automatic potentiometric titrator (for example, AT-610 produced by Kyoto Electronics Manufacturing Co., Ltd.).

Still, let a temperature at a time of measurement be 25±2°C.

A molecular weight of the cyclic amine compound (A) is, from viewpoints of suppressing weight loss in heat treatment to desorb carbon dioxide and further improving the repeated usability, preferably 90 or more, more preferably 120 or more, even more preferably 140 or more, still more preferably 160 or more, yet more preferably 180 or more, even much more preferably 200 or more, still much more preferably 220 or more, and yet much more preferably 240 or more, and from the viewpoint of further improving the amount of carbon dioxide absorbed from the air, preferably 1,000 or less, more preferably 800 or less, even more preferably 600 or less, still more preferably 500 or less, and yet more preferably 400 or less.

A maximum endothermic temperature of the cyclic amine compound (A) as measured by the following method is, from the viewpoints of suppressing the weight loss in the heat treatment to desorb carbon dioxide and further improving the repeated usability, preferably 120°C or more, more preferably 130°C or more, even more preferably 150°C or more, still more preferably 160°C or more, yet more preferably 180°C or more, even much more preferably 200°C or more, and still much more preferably 220°C or more, and from the viewpoint of further improving the amount of carbon dioxide absorbed from the air, preferably 350°C or less.

### (Method)

Heating the cyclic amine compound (A) from 23°C to 350°C at 10°C/minute of the heating rate, measuring a temperature at which an endothermic amount associated with the volatilization of the cyclic amine compound (A) reaches a maximum, and letting this temperature be the maximum endothermic temperature of the cyclic amine compound (A).

An amine value of the cyclic amine compound (A) is, from the viewpoint of further improving the repeated usability and the amount of carbon dioxide absorbed from the air, preferably 400 mgKOH/g or more, more preferably 500 mgKOH/g or more, and even more preferably 550 mgKOH/g or more, and preferably 1,500 mgKOH/g or less, more preferably 1,200 mgKOH/g or less, even more preferably 1,000 mgKOH/g or less, and still more preferably 900 mgKOH/g or less. The amine value indicates an amount of the amine in the compound and is mg number of potassium hydroxide (KOH) of an amount which is equivalent to acid required to neutralize 1 g amount of the compound.

The amine value can be measured by the following method in accordance with JIS K7237-1995.
(1) Dissolving 0.1 g of the cyclic amine compound (A) in 20 mL of acetic acid.
(2) Calculating the amine value by titrating the solution obtained in the above (1) with the 0.1 N perchloric acid-acetic acid solution using the automatic potentiometric titrator (for example, AT-610 produced by Kyoto Electronics Manufacturing Co., Ltd.).

### <Porous Material (B)>

The porous material (B) is preferably one that can support the cyclic amine compound (A) and withstand conditions of capturing carbon dioxide, and includes, for example, at least one selected from the group consisting of silica, alumina, silica-alumina, magnesia, zirconia, zeolite, a zeolite analogous compound, a clay mineral, a natural mineral, activated carbon, a carbon molecular sieve (porous carbon), porous resin (synthetic adsorbent), a metal organic structure, and a waste solid. Among these, as the porous material (B), preferable is at least one selected from the group consisting of the silica and the alumina, more preferable is the silica, and even more preferable is mesoporous silica.

A shape of the porous material (B) is preferably in a particulate form. This can improve a specific surface area of the porous material (B) and improve an amount of the cyclic amine compound (A) supported, thereby further improving the amount of carbon dioxide absorbed in the carbon dioxide absorbent according to the present invention.

A volume median particle size (D₅₀) of the porous material (B) measured by laser diffraction/scattering particle size distribution measurement is preferably 1 µm or more, more preferably 5 µm or more, and even more preferably 10 µm or more, from a viewpoint of improving handling, and preferably 500 µm or less, more preferably 300 µm or less, even more preferably 200 µm or less, still more preferably 180 µm or less, and yet more preferably 160 µm or less, from a viewpoint of further improving the amount of the cyclic amine compound (A) supported.

The specific surface area of the porous material (B) measured by a BET method is preferably 2 m²/g or more, more preferably 10 m²/g or more, even more preferably 100 m²/g or more, still more preferably 200 m²/g or more, yet more preferably 400 m²/g or more, and even much more preferably 600 m²/g or more, from the viewpoint of further improving the amount of the cyclic amine compound (A) supported, and preferably 3,000 m²/g or less, more preferably 1,500 m²/g or less, even more preferably 1,200 m²/g or less, and still more preferably 1,000 m²/g or less, from the viewpoint of improving the handling.

The pore volume of the porous material (B) is preferably 0.1 cm³/g or more, more preferably 0.3 cm³/g or more, and even more preferably 0.5 cm³/g or more, from the viewpoint of further improving the amount of the cyclic amine compound (A) supported, and preferably 5.0 cm³/g or less, more preferably 3.0 cm³/g or less, even more preferably 2.5 cm³/g or less, still more preferably 2.0 cm³/g or less, yet more preferably 1.5 cm³/g or less, and even much more preferably 1.0 cm³/g or less, from the viewpoint of improving the repeated usability.

The specific surface area and the pore volume can be measured, for example, by a specific surface area/pore size distribution measurement analyzer (e.g., product name: ASAP2020, produced by Shimadzu Corporation) using a constant volume method. As a more specific gas adsorption measurement method using the specific surface area/pore size distribution measurement analyzer, for example, a sample is pretreated by heating and vacuum evacuation, and 0.1 g of the sample for measurement is placed in a sample tube. The sample is then heated to 40°C, vacuum evacuated for 6 hours, cooled to room temperature, and mass of the sample is measured. In the measurement, a liquid nitrogen temperature is set and a pressure range is specified, and the specific surface area, the pore volume, and the pore size can be analyzed and calculated from the obtained nitrogen adsorption isotherms.

The porous material (B) may be granulation molded from the particulate porous material by a known method to make pellets, tablets, or other granulated products. The granulation molding method includes, for example, a dry granulation method using a compression molding machine and a wet granulation method using a binder. By using the particulate porous material granulation molded, vibration resistance and abrasion resistance can be imparted, thereby improving physical stability.

The total content of the cyclic amine compound (A) and the porous material (B) in the carbon dioxide absorbent according to the present invention is, from the viewpoint of further improving the repeated usability and the amount of carbon dioxide absorbed from the air, preferably 60% by mass or more, more preferably 70% by mass or more, even more preferably 80% by mass or more, still more preferably 90% by mass or more, yet more preferably 95% by mass or more, even much more preferably 98% by mass or more, and preferably 100% by mass or less, when the total amount of the carbon dioxide absorbent is 100% by mass.

### <Other Components>

The carbon dioxide absorbent according to the present invention may include components other than the cyclic amine compound (A) and the porous material (B) as appropriate to the extent that the effects of the invention are not impaired. The components other than the cyclic amine compound (A) and the porous material (B) include, for example, a deterioration inhibitor, an antifoaming agent, an antioxidant, and a desiccant to remove moisture (magnesium sulfate, molecular sieves, etc.).

A content of water in the carbon dioxide absorbent according to the present invention is, from the viewpoint of further improving the repeated usability and the amount of carbon dioxide absorbed from the air, preferably 10% by mass or less, more preferably 5% by mass or less, even more preferably 1% by mass or less, still more preferably 0.5% by mass or less, yet more preferably 0.1% by mass or less, and even much more preferably 0.01% by mass or less, and it is still much more preferable that the carbon dioxide absorbent according to the present invention is substantially free of water. Here, "substantially free of water" means that water is not intentionally added and does not exclude the presence of a small amount of water as an impurity.

### <Method for Preparing Carbon Dioxide Absorbent>

A method for preparing the carbon dioxide absorbent is not limited, and any known method can be used. For example, the cyclic amine compound (A) and the porous material (B) can be blended, and mixed using a known apparatus to prepare it.

When the carbon dioxide absorbent is in an aspect in which at least some of the cyclic amine compound (A) is supported on the porous material (B), the carbon dioxide absorbent can preferably be prepared by the following method.

First, the cyclic amine compound (A), the porous material (B), and an organic solvent are blended and stirred preferably under a temperature of 5 to 60°C for 1 to 24 hours, to prepare a mixture. The organic solvent is then removed from the mixture obtained by distillation and the like, and the remaining solids are dried under reduced pressure to obtain the carbon dioxide absorbent.

As the organic solvent, from a viewpoint of dispersibility of the cyclic amine compound (A) and porous material (B) and from a viewpoint of easy removal from the carbon dioxide absorbent, preferable is a monovalent alcohol having 4 or less of carbon atoms, and more preferable is at least one selected from the group consisting of methanol, ethanol, and isopropyl alcohol.

### [Method for Capturing Carbon Dioxide]

A method for capturing carbon dioxide (hereinafter simply referred to as "the method of the present invention", too) according to the present invention is characterized by comprising using the above carbon dioxide absorbent. According to the method of the present invention, the amount of carbon dioxide absorbed from a gas containing carbon dioxide can be improved. It also enables to capture carbon dioxide at lower energy, and the repeated usability of the carbon dioxide absorbent is also good.

It is preferable that the method for capturing carbon dioxide according to the present invention comprises a step of bringing the gas containing carbon dioxide into contact with the carbon dioxide absorbent, to cause the carbon dioxide absorbent to absorb carbon dioxide (absorption step).

### <Absorption Step>

The absorption step is a step of bringing the gas containing carbon dioxide into contact with the carbon dioxide absorbent, to cause the carbon dioxide absorbent to absorb the carbon dioxide. A contact method of the carbon dioxide absorbent and the gas can be selected as suitable one depending on morphology of the carbon dioxide absorbent and is not limited. For example, by passing the gas containing carbon dioxide through the carbon dioxide absorbent, spraying the carbon dioxide absorbent in the gas containing carbon dioxide, or locating the carbon dioxide absorbent in the gas containing carbon dioxide, the gas containing carbon dioxide can be brought into contact with the carbon dioxide absorbent.

The gas containing carbon dioxide is not limited, but includes, for example, air, thermal power plant exhaust gas, steel plant exhaust gas, cement plant exhaust gas, chemical plant exhaust gas, bio-fermentation gas, and natural gas. There is a need for capturing carbon dioxide from these gases with particularly energy savings, and the present invention is particularly effective. A concentration of carbon dioxide in the gas, pressure of the gas, and a temperature of the gas are not limited, and the method of the present invention can be applied in gases with a wide range of conditions.

In addition, the gas containing carbon dioxide may also contain acidic gas and the like other than carbon dioxide. The acidic gas includes CO, NOx, and SOx in the exhaust gas, formaldehyde come up from methanol-fueled power generation, and other hydrogen chloride, hydrogen sulfide. When the above gas containing carbon dioxide contains acidic gases and the like other than carbon dioxide, it is preferable that known steps for removing other acidic gases are combined. Specifically, included is an aspect that the method for capturing carbon dioxide of the present invention is applied to the gas containing acidic gases and the like other than carbon dioxide, or an aspect that the method for capturing carbon dioxide of the present invention is applied after removing other acidic gases from the gas containing acidic gases and the like other than carbon dioxide by a known means.

In the absorption step, a temperature when the gas containing carbon dioxide is brought into contact with the carbon dioxide absorbent is preferably 0°C or more and less than 60°C, more preferably 20°C or more and less than 60°C, and even more preferably 30°C or more and less than 60°C, from the viewpoint of improving the amount of carbon dioxide absorbed.

More preferably, the method of the present invention comprises the absorption step of bringing the gas containing carbon dioxide into contact with the carbon dioxide absorbent, to cause the carbon dioxide absorbent to absorb the carbon dioxide, and a desorption step of desorbing carbon dioxide from the carbon dioxide absorbent with carbon dioxide absorbed in the absorption step, wherein the desorption step comprises at least one step selected from the group consisting of the following (I) to (III). By this method, carbon dioxide can be separated and captured from the gas containing carbon dioxide.
(I) a step of subjecting the carbon dioxide absorbent with carbon dioxide absorbed to a reduced pressure condition;
(II) a step of bringing an inert gas not containing carbon dioxide into contact with the carbon dioxide absorbent with carbon dioxide absorbed; and
(III) a step of heating the carbon dioxide absorbent with carbon dioxide absorbed.

### <Desorption Step>

A desorption step is a step of desorbing carbon dioxide from the carbon dioxide absorbent with carbon dioxide absorbed in the absorption step. The method for desorbing carbon dioxide from the carbon dioxide absorbent with carbon dioxide absorbed preferably includes a method comprising at least one step selected from the group consisting of the above (I) to (III). Two or more of the steps (I) to (III) may be combined.

In the (I) step of subjecting the carbon dioxide absorbent with carbon dioxide absorbed to the reduced pressure condition (hereinafter referred to as "Step (I)", too), the reduced pressure condition is preferably 10 kPa or less, more preferably 5 kPa or less, even more preferably 1 kPa or less, from a viewpoint of improving carbon dioxide separating and capturing efficiency. In addition, from a viewpoint of inhibiting volatilization of the cyclic amine compound (A) in the carbon dioxide absorbent, it is preferably 0.1 kPa or more.

In Step (I), a temperature when the carbon dioxide absorbent is subjected to the reduced pressure condition is not limited, but from the viewpoint of inhibiting the volatilization of the cyclic amine compound (A) in the carbon dioxide absorbent, it is preferably less than 50°C, more preferably 45°C or less. In addition, from the viewpoint of improving the carbon dioxide separating and capturing efficiency, it is preferably 0°C or more, and more preferably 10°C or more.

In the (II) step of bringing the inert gas not containing carbon dioxide into contact with the carbon dioxide absorbent with carbon dioxide absorbed (hereinafter referred to as "Step (II)", too), reducing a partial pressure of carbon dioxide allows to promote the desorption of carbon dioxide. The inert gas not containing carbon dioxide includes nitrogen, helium, argon, and one or more of these gases can be used. Above all, from the viewpoint of improving the carbon dioxide separating and capturing efficiency, the inert gas not containing carbon dioxide is preferably at least one selected from the group consisting of the nitrogen and the argon.

In Step (II), the method for bringing the inert gas not containing carbon dioxide into contact with the carbon dioxide absorbent includes the same method as the contact method described in the absorption step.

In Step (II), a temperature when the inert gas not containing carbon dioxide is brought into contact with the carbon dioxide absorbent is not limited, and heating as specified in the step (III) may be simultaneously performed in Step (II), or may be at a temperature of room temperature or less. From the viewpoint of inhibiting the volatilization of the cyclic amine compound (A) in the carbon dioxide absorbent, the temperature is preferably less than 50°C, and more preferably 45°C or less. In addition, from the viewpoint of improving the carbon dioxide separating and capturing efficiency, the temperature is preferably 0°C or more, and more preferably 10°C or more.

A heating temperature in the (III) step of heating the carbon dioxide absorbent with carbon dioxide absorbed (hereinafter referred to as "Step (III)", too) is preferably 50°C or more and 120°C or less, more preferably 55°C or more and 110°C or less, and even more preferably 60°C or more and 100°C or less, from the viewpoint of improving the carbon dioxide separating and capturing efficiency.

The heating in Step (III) can be performed by a known method using apparatus equipped with a heating means. A heating method includes, for example, heating with steam or a heating medium, hot air heating, electromagnetic wave heating, ultrasonic heating, and induction heating.

The carbon dioxide absorbent and the carbon dioxide which are separated in the desorption step can be captured separately and reused.

### [Carbon Dioxide Separation and Capture Apparatus]

The carbon dioxide separation and capture apparatus of the present invention (hereinafter referred to as "the apparatus of the present invention", too) comprises an absorption apparatus comprising a mechanism for bringing the gas containing carbon dioxide into contact with the carbon dioxide absorbent, to cause the carbon dioxide absorbent to absorb the carbon dioxide, and a desorption apparatus comprising a mechanism for desorbing carbon dioxide from the carbon dioxide absorbent with carbon dioxide absorbed.

The apparatus of the present invention will be described with reference to Fig. 1. Fig. 1 is a schematic diagram showing an embodiment of a carbon dioxide separation and capture apparatus 100 of the present invention, in which 1 denotes the absorption apparatus and 2 denotes the desorption apparatus in Fig. 1.

### <Absorption Apparatus>

The absorption apparatus 1 in the carbon dioxide separation and capture apparatus 100 is equipped with the carbon dioxide absorbent and comprises a mechanism for bringing the gas containing carbon dioxide into contact with the carbon dioxide absorbent, to cause the carbon dioxide absorbent to absorb the carbon dioxide.

The absorption apparatus 1 is not limited, as long as it has configuration such that the gas containing carbon dioxide are brought into contact with the carbon dioxide absorbent, depending on the morphology of the carbon dioxide absorbent. As shown in Fig. 1, for example, the absorption apparatus 1 can be equipped with an absorbent holding part 12 that holds a carbon dioxide absorbent 12a inside a reaction column 11 and can be further equipped with a gas supplying part 13 that supplies the gas containing carbon dioxide to the absorbent holding part 12. In addition, the absorption apparatus 1 can be also equipped with an absorbent discharging part (not shown) that discharges the carbon dioxide absorbent 12a held in the absorbent holding part 12 and an absorbent supplying part (not shown) that supplies a new carbon dioxide absorbent to the absorbent holding part 12, from a viewpoint of discharging the carbon dioxide absorbent with carbon dioxide absorbed from the absorbent holding part 12 and supplying a new carbon dioxide absorbent.

The absorption apparatus 1 may further comprise a heating and cooling mechanism to adjust the temperature when the gas containing carbon dioxide is brought into contact with the carbon dioxide absorbent. It may also comprise a carbon dioxide concentration measuring mechanism so that a carbon dioxide concentration in the gas can be measured. Furthermore, the absorption apparatus 1 may comprise a pressurizing and depressurizing mechanism to adjust pressure when the gas containing carbon dioxide is brought into contact with the carbon dioxide absorbent.

The carbon dioxide separation and capture apparatus 100 may have a connecting part 3 for supplying the carbon dioxide absorbent with carbon dioxide absorbed in the absorption apparatus 1 to the desorption apparatus 2.

A means of supplying the carbon dioxide absorbent with carbon dioxide absorbed from the absorption apparatus 1 to the desorption apparatus 2 is not limited, and the absorption apparatus 1 operated for a certain period of time may be stopped once, and the carbon dioxide absorbent in the reaction column 11 equipped in the absorption apparatus 1 may be supplied to the desorption apparatus 2 together. Alternatively, from the absorbent holding part 12 of the absorption apparatus 1, the carbon dioxide absorbent may be supplied continuously or interruptedly to the desorption apparatus 2 by utilizing the connecting part 3.

### <Desorption Apparatus>

The desorption apparatus 2 in the carbon dioxide separation and capture apparatus 100 comprises a mechanism for desorbing carbon dioxide from the carbon dioxide absorbent by heating the carbon dioxide absorbent with carbon dioxide absorbed in the absorption apparatus 1. The desorption apparatus 2 is not limited as long as it comprises a mechanism for desorbing carbon dioxide from the carbon dioxide absorbent with carbon dioxide absorbed in the absorption apparatus 1, but it is preferable that it comprises a mechanism to perform at least one step of the above (I) to (III). Examples of the mechanism include a depressurizing mechanism, an inert gas supplying mechanism, and a heating mechanism.

As shown in Fig. 1, for example, the desorption apparatus 2 can be equipped with an absorbent holding part 22 that holds a carbon dioxide absorbent 22a with carbon dioxide absorbed inside a reaction column 21, and further equipped with a gas discharging part 23 that discharges carbon dioxide desorbed from the carbon dioxide absorbent. The desorption apparatus 2 also comprises at least one mechanism (not shown) selected from the group consisting of a depressurizing mechanism for bringing the inside of the reaction column 21 under a reduced pressure condition, an inert gas supplying mechanism for supplying the inert gas to the absorbent holding part 22, and a heating mechanism for heating the absorbent holding part 22.

In addition to the depressurizing mechanism, the inert gas supplying mechanism, and the heating mechanism described above, the desorption apparatus 2 may comprise the carbon dioxide concentration measuring mechanism or the like, in the same manner as the absorption apparatus 1.

The carbon dioxide absorbent after carbon dioxide is desorbed in the desorption apparatus 2 can also be reused by supplying it back into the absorption apparatus 1 again from an absorbent discharging part 24 for supplying the carbon dioxide absorbent after carbon dioxide is desorbed to the absorption apparatus 1.

The carbon dioxide separation and capture apparatus 100 may further comprise a capturing apparatus for capturing carbon dioxide desorbed. Still, here, the carbon dioxide captured may be used for agricultural applications such as a petroleum promoted capturing method and a plant factory; industrial gas applications such as beverages and welding; chemical synthesis applications; and carbon dioxide capture and storage (CCS) applications. Prior to use in these applications, the carbon dioxide captured may also be concentrated.

### Examples

Hereinafter, the present invention will be described with reference to examples, but the present invention is not limited to the scope of the examples. In the present examples, various measurements and evaluations were performed by the following methods.

### (Acid Dissociation Constant (pKa) of Amine Compound)

The acid dissociation constant of the amine compound was determined by the following method.
(1) Dissolving 0.2 g of the amine compound in 30 mL of the purified water.
(2) Calculating the acid dissociation constant (pKa) by titrating the solution obtained in the above (1) with the 0.1 N perchloric acid-acetic acid solution using the automatic potentiometric titrator (AT-610 produced by Kyoto Electronics Manufacturing Co., Ltd.).

Still, let the temperature at the time of measurement be 25±2°C.

### (Amine Value of Amine Compound)

The amine value was measured by the following measurement method in accordance with JIS K7237-1995.
(1) Dissolving 0.1 g of the amine compound in 20 mL of the acetic acid.
(2) Calculating the amine value by titrating the solution obtained in the above (1) with the 0.1 N perchloric acid-acetic acid solution using the automatic potentiometric titrator (AT-610 produced by Kyoto Electronics Manufacturing Co., Ltd.).

### (Maximum Endothermic Temperature of Amine Compound)

DSC measurements were performed on the amine compounds to measure the maximum endothermic temperature of the amine compounds as follows. First, a differential scanning calorimetry was performed on the amine compounds using a differential thermogravimetric analyzer (Product Name: DTG-60 produced by Shimadzu Corporation) under conditions of 23 to 350°C of a measurement temperature range, 10°C/minute of a heating rate, and a nitrogen atmosphere. From the DSC curve obtained thereby, the temperature at which the endothermic amount associated with the volatilization of the amine compound reached the maximum was calculated, and the temperature thereof was used as the maximum endothermic temperature of the amine compound.

### (Maximum Carbon Dioxide (CO₂) Release Temperature of Amine Compound)

A carbon dioxide concentration meter and a petri dish were placed inside an openable desiccator (Internal Dimension: 370 mm x 260 mm x 272 mm). The amine compound (5 mmol) was then added to the petri dish in the desiccator, a door was immediately closed, and the amine compound was stood in the desiccator for 24 hours under an air environment of 23°C and 50% RH. An initial concentration of carbon dioxide was adjusted to about 400 ppm.

Then, the amine compound was removed from the desiccator to obtain the amine compound with carbon dioxide absorbed. The DSC measurement was performed on the amine compound with carbon dioxide absorbed as follows, to measure the maximum carbon dioxide release temperature of the amine compound. First, the differential scanning calorimetry was performed on the amine compound using the differential thermogravimetric analyzer (Product Name: DTG-60 produced by Shimadzu Corporation) under the conditions of 23 to 250°C of a measurement temperature range, 10°C/minute of the heating rate, and the nitrogen atmosphere. From the DSC curve obtained thereby, the temperature at which the endothermic amount associated with the desorption of carbon dioxide reached the maximum was calculated, and the temperature thereof was used as the maximum carbon dioxide release temperature of the amine compound.

### (Specific Surface Area and Pore Volume of Porous Material)

The specific surface area and the pore volume of the porous material were measured by the specific surface area/pore size distribution measurement analyzer (product name: ASAP2020, produced by Shimadzu Corporation).

### (Volume Median Particle Size (D₅₀) of Porous Material)

Using a laser diffraction/scattering particle size distribution analyzer (produced by Malvern Panalytical Ltd., product name "LMS-200e"), particle distribution of the porous material was measured.

Then, the particle diameter corresponding to 50% of a cumulative volume frequency calculated from the smaller particle diameter of the particle distribution was taken as the volume median particle size (D₅₀) of the porous material.

### (Evaluation 1 of Carbon Dioxide Absorption Capacity)

To a glass container with nitrogen replacement, 300 mg of the amine compound, 10 g of methanol, and 300 mg of the porous material were added and stirred for 10 hours to homogenize them. The resulting mixture was then placed under a circumstance of 40°C and 100 hPa, to distil the methanol off, followed by vacuum drying at room temperature (23°C) for 24 hours to obtain the carbon dioxide absorbent.

Then, 15 mg of the carbon dioxide absorbent obtained was set in a differential thermogravimetric analyzer (product name: EXSTER TGD6200, produced by Hitachi High-Tech Corporation) and stood at 45°C under a dry air environment for 6 hours, and an increased amount in mass of the carbon dioxide absorbent was measured. Here, as a gas used at measurements, air (flow rate: 200 ml/min) and nitrogen (flow rate: 200 ml/min) were used at absorbing carbon dioxide and desorbing carbon dioxide, respectively. From the increased amount in the mass of the carbon dioxide absorbent, the amount of carbon dioxide absorbed of the carbon dioxide absorbent was calculated (1st time). A unit of the amount of carbon dioxide absorbed in Table 1 is an amount of carbon dioxide (mg) absorbed per 1 g of the carbon dioxide absorbent.

After the 1st time evaluation of the carbon dioxide absorption capacity was completed, the carbon dioxide absorbent was removed from the analyzer and the carbon dioxide absorbent with carbon dioxide absorbed was heated at 125°C for 30 minutes to desorb the carbon dioxide absorbed, regenerating the carbon dioxide absorbent.

Then, on the carbon dioxide absorbent regenerated, the above evaluation of the carbon dioxide absorption capacity was performed again, to measure an amount of carbon dioxide absorbed (2nd time).

Then, the carbon dioxide absorbent was removed from the analyzer and the carbon dioxide absorbent with carbon dioxide absorbed was heated at 125°C for 30 minutes to desorb the carbon dioxide absorbed, regenerating the carbon dioxide absorbent again.

Then, on the carbon dioxide absorbent regenerated, the above evaluation of the carbon dioxide absorption capacity was performed again, to measure the amount of carbon dioxide absorbed (3rd time).

Here, the retention rate of the amount of carbon dioxide absorbed was also calculated based on the amount of carbon dioxide absorbed of the 1st time.

In Examples and Comparative Examples, as the amine compound and the porous materials, the following were used.

### (Amine Compounds)

1,3-BAC-BisAP: a hydrogenated product of a reaction adduct of 1:2 (molar ratio) of 1,3-bis(aminomethyl)cyclohexane and acrylonitrile (prepared according to the following Synthetic Example 1)
1,3-BAC-TetraAP: a hydrogenated product of a reaction adduct of 1:4 (molar ratio) of 1,3-bis(aminomethyl)cyclohexane and acrylonitrile (prepared according to the following Synthetic Example 2)
TETA: triethylenetetramine (produced by Tokyo Chemical Industry Co., Ltd.), an acyclic amine compound having 50% by mol of the primary amino groups with respect to the total amino groups
AEP: 2-aminoethylpiperazine (produced by Tokyo Chemical Industry Co., Ltd.), a cyclic amine compound having 33% by mol of the primary amino groups with respect to the total amino groups

### (Synthesis Example 1: Preparation of 1,3-BAC-BisAP)

(1) To the round-bottomed flask having the inner volume of 100 mL equipped with the stirrer, the thermometer, the argon inlet tube, the dropping funnel and the cooling tube, 10.0 g of 1,3-bis(aminomethyl)cyclohexane (produced by Mitsubishi Gas Chemical Company, Inc.) and 20.0 g of 2-propanol (produced by FUJIFILM Wako Pure Chemical Corporation) were added, and after thorough stirring under a flow of argon, 7.5 g of acrylonitrile (produced by Sigma-Aldrich Co. LLC) was added dropwise over a period of 10 minutes. After completion of the dropping, a temperature was raised to 65°C and held for 1 hour, then cooled to room temperature to obtain a reaction liquid (1).
(2) To a tubular vertical hydrogenation reactor (made of glass, inner diameter of 10 mmφ), 7.0 g of hydrogenation catalyst (three-leaf type, diameter of 1.2 mmφ, produced by Johnson Matthey Japan G.K.; HTCCo2000) having 15% by mass of a cobalt content was charged, and after held at 120°C for 1 hour under a flow of hydrogen, raised to 240°C and held for 4 hours or longer for reduction and activation. After cooling, 14.8 g of 2-propanol, the above catalyst and all of the reaction liquid (1) were added to an autoclave (capacity of 150 mL, material: SUS316L) equipped with a stirrer and a heater, and a gas phase portion was replaced with hydrogen. After pressurizing to 3.5 MPaG with the hydrogen, the temperature was started to be raised while stirring, and a liquid temperature was brought to 80°C in 20 minutes, then the pressure was adjusted to 8.0 MPaG. The reaction was then continued for 3 hours while hydrogen was fed as needed to maintain the pressure at 8.0 MPaG under the condition of 80°C of the liquid temperature. The reaction liquid was completely concentrated under vacuum to obtain 17.5 g of 1,3-BAC-BisAP.

1,3-BAC-BisAP has four amino groups in the molecule, and two of the primary amino groups. In other words, 1,3-BAC-BisAP has 50% by mol of the primary amino groups with respect to the total amino groups.

### (Synthesis Example 2: Preparation of 1,3-BAC-TetraAP)

(1) To the round-bottomed flask having the inner volume of 300 mL equipped with the stirrer, the thermometer, the argon inlet tube, the dropping funnel, and the cooling tube, 10 g of 1,3-bis(aminomethyl)cyclohexane (produced by Mitsubishi Gas Chemical Company, Inc.), 100.0 g of 2-propanol (produced by FUJIFILM Wako Pure Chemical Corporation), and 100.0 g of distilled water were added, and after thorough stirring under the flow of argon, 18.6 g of acrylonitrile (produced by Sigma-Aldrich Co. LLC) was added dropwise over a period of 10 minutes. After completion of the dropping, a temperature was raised to 50°C and held for 6 hours, then the temperature was raised to 80°C and additional 37.2 g of the acrylonitrile was added dropwise, and cooled to room temperature after holding for 18 hours, and the solvent was distilled off to obtain 25 g of acrylonitrile 4-adduct of the 1,3-bis(aminomethyl)cyclohexane.
(2) To the autoclave (capacity of 150 mL, material: SUS316L) equipped with the stirrer and the heater, 0.75 g of hydrogenation catalyst (three-leaf type, diameter of 1.2 mmφ, produced by Johnson Matthey Japan G.K.; HTCCo2000) having 15% by mass of a cobalt content, 60.0 g of 2-propanol, and 1.5 g of the acrylonitrile 4-adduct of the 1,3-bis(aminomethyl)cyclohexane were added, and the gas phase portion was replaced with hydrogen. The temperature was started to be raised while stirring, and after the liquid temperature reached 80°C, the reaction was continued for 5 hours under the condition of 80°C while supplying hydrogen as needed for holding the pressure at 6.0 MPa, after pressurizing to 6.0 MPa with hydrogen. By distilling the solvent off from the reaction liquid, 1.5 g of 1,3-BAC-TetraAP was obtained.

The 1,3-BAC-TetraAP has six amino groups in the molecule, and four of the primary amino groups. In other words, 1,3-BAC-TetraAP has 66.7% by mol of the primary amino groups with respect to the total amino groups.

### (Porous Material)

### Mesoporous Silica SBA15 (produced by Merck KGaA)

The specific surface area by the BET method: 800 m²/g, the volume median particle size (D₅₀): 100 µm, the pore volume: 0.8 cm³/g

### Fumed Silica QS-40 (produced by Tokuyama Corporation)

Hydrophilic fumed silica, the specific surface area by the BET method: 370 m²/g, the volume median particle size (D₅₀): 10 µm

### Fumed Silica RX-300 (produced by NIPPON AEROSIL CO., LTD.)

Hydrophobically treated fumed silica, the specific surface area by the BET method: 300 m²/g, the volume median particle size (D₅₀): 20 µm

### Mesoporous Alumina PULAROX (produced by SAZOL LTD.)

The specific surface area by the BET method: 150 m²/g, the volume median particle size (D₅₀): 35 µm, the pore volume: 0.9 cm³/g

### Porous Carbon MJ(4)030 (produced by Toyo Tanso Co., Ltd.)

The specific surface area by the BET method: 670 m²/g, the volume median particle size (D₅₀): 5 µm, the pore volume: 1.7 cm³/g

### Synthetic Adsorbent DIAION HP-20 (produced by Mitsubishi Chemical Corporation)

The specific surface area by the BET method: 590 m²/g, the volume median particle size (D50): 250 µm, the pore volume: 1.3 cm³/g

### (Examples 1 to 2 and Comparative Examples 1 to 2)

In Example 1 and Comparative Example 1, each of the above evaluations was respectively performed using the amine compound and the porous material shown in Table 1. The results obtained are shown in Table 1.

**Table 1**

| | Amine Compound | | | | | | | Porous Material (B) | Evaluation 1 of CO₂ Absorption Capacity | | | | | |
|---|---|---|---|---|---|---|---|---|---|---|---|---|---|---|
| | Name | Structural Formula | Molecular Weight [-] | Maximum CO₂ Release Temperature [°C] | Maximum Endothermic Temperature [°C] | Amine Value [mgKOH/g] | pKa | Name | Amount of CO₂ Absorbed [mg-CO₂/g] | | | Retention Rate of Amount of CO₂ Absorbed [%] | | |
| | | | | | | | | | 1st Time | 2nd Time | 3rd Time | 1st Time | 2nd Time | 3rd Time |
| Example 1 | 1,3-BAC -BisAP | 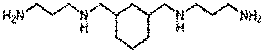 | 256.4 | 97.8 | 246.7 | 844 | 9.5 | Mesoporous Silica SBA15 | 31.4 | 31.4 | 30.9 | 100.0 | 100.0 | 98.2 |
| Example 2 | 1,3-BAC -tetraAP | 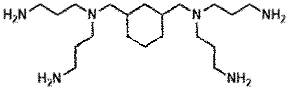 | 370.6 | 102.3 | 298.2 | 890 | 9.8 | Mesoporous Silica SBA15 | 35.5 | 35.5 | 35.1 | 100.0 | 100.0 | 98.8 |
| Comparative Example 1 | TETA | 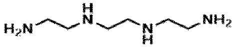 | 146.2 | 69.5 | 184.1 | 1535 | 10.3 | Mesoporous Silica SBA15 | 47.6 | 37.0 | 24.6 | 100.0 | 77.6 | 51.7 |
| Comparative Example 2 | AEP | 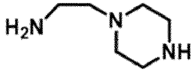 | 129.2 | 81.9 | 118.4 | 1154 | 10.1 | Mesoporous Silica SBA15 | 16.5 | 1.6 | 1.5 | 100.0 | 9.7 | 9.1 |

From Table 1, it can be seen that the carbon dioxide absorbents of Examples 1 to 2 containing the cyclic amine compound (A) having 35% by mol or more of the primary amino group with respect to the total amino groups and the porous material (B) show a small decrease in the amount of carbon dioxide absorbed even after repeated uses. In other words, it can be seen that the carbon dioxide absorbent of the present invention can be improved in the repeated usability. In contrast, it can be seen that the carbon dioxide absorbents of Comparative Examples 1 to 2 show a large decrease in the amount of carbon dioxide absorbed if used repeatedly, indicating inferior in terms of the repeated usability.

### (Examples 3 to 9, Comparative Examples 3 to 4: Evaluation of Carbon Dioxide Absorption Capacity under Practical Application Conditions)

The carbon dioxide absorbents are used under practical application conditions where carbon dioxide is captured from a gas mixture containing moisture in addition to carbon dioxide such as under an air environment that includes outdoor air and indoor space or combustion exhaust gas. Therefore, in Examples 3 to 9 and Comparative Examples 3 to 4, the amount of carbon dioxide absorbed and the amount of carbon dioxide desorbed under humidity-controlled conditions, taking into account the effect of moisture in the gas, were measured using a catalyst analyzer (BELCATII; produced by MicrotracBELL Corporation) by a method described later, to evaluate the carbon dioxide absorption capacity of the carbon dioxide absorbent.

### (Evaluation 2 of Carbon Dioxide Absorption Capacity: Heating Cycle Evaluation of Carbon Dioxide Absorbent)

(1) To the glass container with nitrogen replacement, 300 mg of the amine compound, 10 g of methanol, and 300 mg of the porous material were added and stirred for 10 hours to homogenize them. The resulting mixture was then placed under the circumstance of 40°C and 100 hPa, to distil the methanol off, followed by the vacuum drying at room temperature (23°C) for 24 hours to obtain the carbon dioxide absorbent.
(2) Then, 200 mg of the carbon dioxide absorbent obtained was charged into a reaction tube of the above catalyst analyzer, and heated at 100°C for 1 hour in a nitrogen stream (flow rate: 100 ml/min) to perform dry and degas pretreatment, and then the reaction tube was kept at 40°C. Then, the introducing gas was switched to a 400 ppm of carbon dioxide/nitrogen gas mixture (total flow rate: 1,000 ml/min) humidity-controlled to 40°C and 40% RH (absorption step), and at the same time, a change in an outlet gas composition of the catalyst analyzer over time was measured by a gas mass spectrometer (BELMASS; produced by MicrotracBELL Corporation), to obtain a breakthrough curve. After the amount of carbon dioxide absorbed reached saturation, the reaction tube was heated to 80°C along with switching the introducing gas to nitrogen (flow rate: 500 ml/min) (desorption step), and the change in the outlet gas composition of the catalyst analyzer over time was subsequently measured by the gas mass spectrometer.
   The amount of carbon dioxide absorbed in the carbon dioxide absorbent was calculated from, the time from a start of absorption until saturation was reached, and an accumulated value of an outlet concentration change of carbon dioxide, and is shown in Table 2 (1st time evaluation of the carbon dioxide absorption capacity). The amount of carbon dioxide desorbed from the carbon dioxide absorbent was calculated from, the time from when the introducing gas was switched to nitrogen until carbon dioxide was almost undetectable from the outlet side, and the accumulated value of the outlet concentration change of carbon dioxide.
(3) After the 1st time evaluation of the carbon dioxide absorption capacity was completed, with respect to the carbon dioxide absorbent regenerated by performing the above desorption step, an operation of the above (2) was further repeated 9 times, resulting in a total of 10 times of carbon dioxide absorption and desorption steps. Based on the amount of carbon dioxide absorbed in the 1st time evaluation, the retention rate of the amount of carbon dioxide absorbed in the 5th and 10th evaluations were calculated.

### (Evaluation 3 of Carbon Dioxide Absorption Capacity: Reduced Pressure Cycle Evaluation of Carbon Dioxide Absorbent)

(1) After 100 mg of the carbon dioxide absorbent prepared by the same method as described above was weighed and charged in the reaction tube of the above catalyst analyzer, a temperature of the absorbent was held at 60°C to perform pretreatment by pressure reducing exhaust for 1 hour. Then, the reaction tube was kept at 40°C, and the introducing gas was switched to the 400 ppm of carbon dioxide/nitrogen gas mixture (total flow rate: 1,000 ml/min) humidity-controlled to 40°C and 40% RH (absorption step), and at the same time, a change in the outlet gas composition of the catalyst analyzer over time was measured by the gas mass spectrometer (BELMASS; produced by MicrotracBELL Corporation), to obtain a breakthrough curve. The amount of carbon dioxide absorbed in the carbon dioxide absorbent was calculated from, the time from the start of absorption until the saturation was reached, and the accumulated value of the outlet concentration change of carbon dioxide, and is shown in Table 3 (1st time evaluation of the carbon dioxide absorption capacity).
(2) The amount of carbon dioxide desorbed due to the reduced pressure in the carbon dioxide absorbent was defined as the amount of carbon dioxide absorbed in a case of using the carbon dioxide absorbent after desorbing carbon dioxide, since the amount of carbon dioxide equivalent to the carbon dioxide desorbed due to the reduced pressure would be absorbed afterward. Specifically, the absorbent with carbon dioxide absorbed in the above (1) was kept at 40°C and subjected to the pressure reduced exhaust as it is for 30 minutes by a manual operation using a vacuum pump (desorption step), and then, again, by the same method as the above (1), the 400 ppm of carbon dioxide/nitrogen gas mixture was introduced, and the changes in the outlet gas composition over time were obtained by measuring them by the gas mass spectrometer (BELMASS; produced by MicrotracBELL Corporation). Still, the attained vacuum at a time of pressure reduction for 30 minutes was 0.5 kPa.
(3) After the 1st time evaluation of the carbon dioxide absorption capacity was completed, with respect to the carbon dioxide absorbent regenerated by performing the desorption step described in the above (2), the step of the above (1) and the desorption step were further repeated 9 more times, resulting in the total of 10 times of the carbon dioxide absorption and desorption steps. Based on the amount of carbon dioxide absorbed in the 1st time evaluation, the retention rates of the amount of carbon dioxide absorbed in the 5th time and 10th time evaluations were calculated.

Still, a unit of the amount of carbon dioxide absorbed in Tables 2 and 3 is the amount of carbon dioxide absorbed (mg) per 1 g of the carbon dioxide absorbent.

**Table 2**

| | Amine Compound | Porous Material (B) | Absorption Step | Desorption Step | Evaluation 2 of CO₂ Absorption Capacity | | | | | |
|---|---|---|---|---|---|---|---|---|---|---|
| | | | | | Amount of CO₂ Absorbed [mg-CO₂/g] | | | Retention Rate of Amount of CO₂ Absorbed [%] | | |
| | Name | Name | CO₂ Absorption Condition | CO₂ Desorption Condition | 1st Time | 5th Time | 10th Time | 1st Time | 5th Time | 10th Time |
| Example 3 | 1,3-BAC-BisAP | Mesoporous Silica SBA15 | 400 ppm CO₂/N₂ Gas Mixture, 40°C, 40%RH, 2 hours | 80°C/Normal Pressure, 30 minutes | 74.1 | 72.7 | 71.5 | 100 | 98.1 | 96.5 |
| Example 4 | 1,3-BAC-tetraAP | Mesoporous Silica SBA15 | | | 80.5 | 79.8 | 78.9 | 100 | 99.1 | 98.0 |
| Example 5 | 1,3-BAC-BisAP | Fumed Silica QS-40 | | | 58.7 | 57.3 | 56.5 | 100 | 97.6 | 96.3 |
| Example 6 | 1,3-BAC-BisAP | Fumed Silica RX-300 | | | 57.2 | 56.1 | 55.0 | 100 | 98.1 | 96.2 |
| Example 7 | 1,3-BAC-BisAP | Mesoporous Alumina PULAROX | | | 48.8 | 47.6 | 46.2 | 100 | 97.5 | 94.7 |
| Example 8 | 1,3-BAC-BisAP | Porous Carbon MJ(4)030 | | | 48.2 | 45.8 | 44.9 | 100 | 95.0 | 93.2 |
| Example 9 | 1,3-BAC-BisAP | Synthetic Adsorbent DIAION HP-20 | | | 51.3 | 50.3 | 49.5 | 100 | 98.1 | 96.5 |
| Comparative Example 3 | TETA | Mesoporous Silica SBA15 | | | 76.3 | 54.3 | 35.7 | 100 | 71.2 | 46.8 |
| Comparative Example 4 | AEP | Mesoporous Silica SBA15 | | | 31.2 | 1.5 | 1.4 | 100 | 4.8 | 4.5 |

**Table 3**

| | Amine Compound | Porous Material (B) | Absorption Step | Desorption Step | Evaluation 3 of CO₂ Absorption Capacity | | | | | |
|---|---|---|---|---|---|---|---|---|---|---|
| | | | | | Amount of CO₂ Absorbed [mg-CO₂/g] | | | Retention Rate of Amount of CO₂ Absorbed [%] | | |
| | Name | Name | CO₂ Absorption Condition | CO₂ Desorption Condition | 1st Time | 5th Time | 10th Time | 1st Time | 5th Time | 10th Time |
| Example 3 | 1,3-BAC-BisAP | Mesoporous Silica SBA15 | 400 ppm CO₂/N₂ Gas Mixture, 40°C, 40%RH, 2 hours | 40°C/0.5 kPa, 30 minutes | 74.1 | 73.5 | 72.5 | 100 | 99.2 | 97.8 |
| Example 4 | 1,3-BAC-tetraAP | Mesoporous Silica SBA15 | | | 80.5 | 80.1 | 79.5 | 100 | 99.5 | 98.8 |
| Example 5 | 1,3-BAC-BisAP | Fumed Silica QS-40 | | | 58.7 | 57.3 | 56.3 | 100 | 97.6 | 95.9 |
| Example 6 | 1,3-BAC-BisAP | Fumed Silica RX-300 | | | 57.2 | 56.1 | 55.9 | 100 | 98.1 | 97.7 |
| Example 7 | 1,3-BAC-BisAP | Mesoporous Alumina PULAROX | | | 48.8 | 47.9 | 46.9 | 100 | 98.2 | 96.1 |
| Example 8 | 1,3-BAC-BisAP | Porous Carbon MJ(4)030 | | | 48.2 | 45.6 | 44.1 | 100 | 94.6 | 91.5 |
| Example 9 | 1,3-BAC-BisAP | Synthetic Adsorbent DIAION HP-20 | | | 51.3 | 50.6 | 49.8 | 100 | 98.6 | 97.1 |
| Comparative Example 3 | TETA | Mesoporous Silica SBA15 | | | 76.2 | 56.9 | 43.7 | 100 | 74.7 | 57.3 |
| Comparative Example 4 | AEP | Mesoporous Silica SBA15 | | | 31.2 | 16.5 | 16.1 | 100 | 52.9 | 51.6 |

From Tables 2 and 3, it can be seen that the carbon dioxide absorbents of Examples 3 to 9 containing the cyclic amine compounds (A) having 35% by mol or more of the primary amino group with respect to the total amino groups and the porous material (B) show a small decrease in the amount of carbon dioxide absorbed and good in the repeated usability even if they are repeatedly used by using the gas mixture containing moisture in addition to carbon dioxide. In contrast, it can be seen that the carbon dioxide absorbents of Comparative Examples 3 to 4, show a large decrease in the amount of carbon dioxide absorbed and poor repeated usability if they are repeatedly used.

### Industrial Applicability

According to the present invention, the carbon dioxide absorbent having improved repeated usability, the method for capturing carbon dioxide using the carbon dioxide absorbent, and the carbon dioxide separation and capture apparatus can be provided.

### Reference Signs List

- 100: carbon dioxide separation and capture apparatus
- 1: absorption apparatus
- 2: desorption apparatus
- 3: connecting part
- 11, 21: reaction column
- 12, 22: absorbent holding part
- 12a: carbon dioxide absorbent
- 13: gas supplying part
- 21: reaction column
- 22a: carbon dioxide absorbent with carbon dioxide absorbed
- 23: gas discharging part
- 24: absorbent discharging part

## Claims

1. A carbon dioxide absorbent comprising a cyclic amine compound (A) and a porous material (B), wherein the cyclic amine compound (A) has 35% by mol or more of a primary amino group with respect to total amino groups.

2. The carbon dioxide absorbent according to claim 1, wherein at least some of the cyclic amine compound (A) is supported on the porous material (B).

3. The carbon dioxide absorbent according to claim 1 or 2, wherein the porous material (B) comprises at least one selected from the group consisting of silica and alumina.

4. The carbon dioxide absorbent according to any one of claims 1 to 3, wherein the porous material (B) is in a particulate form.

5. The carbon dioxide absorbent according to claim 4, wherein a volume median particle size (D₅₀) of the porous material (B) as measured by laser diffraction/scattering particle size distribution measurement is 1 µm or more and 500 µm or less.

6. The carbon dioxide absorbent according to any one of claims 1 to 5, wherein a specific surface area of the porous material (B) as measured by a BET method is 2 m²/g or more and 3,000 m²/g or less.

7. The carbon dioxide absorbent according to any one of claims 1 to 6, wherein a pore volume of the porous material (B) is 0.1 cm³/g or more and 5.0 cm³/g or less.

8. The carbon dioxide absorbent according to any one of claims 1 to 7, wherein a content of the cyclic amine compound (A) is 0.1 parts by mass or more and 1,000 parts by mass or less with respect to 100 parts by mass of the porous material (B).

9. The carbon dioxide absorbent according to any one of claims 1 to 8, wherein the cyclic amine compound (A) comprises at least one selected from the group consisting of an amine compound (a1) represented by the following formula (1) and an amine compound (a2) having a heterocyclic structure selected from the group consisting of an oxygen-containing heterocyclic structure and a sulfur-containing heterocyclic structure, 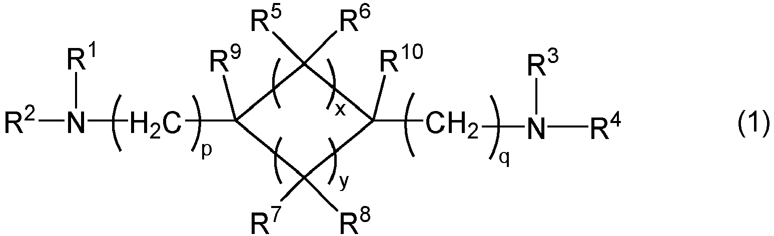 wherein R¹ to R⁴ each independently represent a hydrogen atom, or a hydrocarbon group having 1 or more and 10 or less carbon atoms and optionally having at least one substituent selected from the group consisting of an amino group, a cyano group and a phenyl group; R⁵ to R¹⁰ each independently represent a hydrogen atom or a hydrocarbon group having 1 or more and 4 or less carbon atoms; x and y each independently represent an integer of 0 or more and 6 or less; x + y is 1 or more and 6 or less; p and q each independently represent an integer of 0 or more and 4 or less; and at least one of p and q is 1 or more.

10. The carbon dioxide absorbent according to any one of claims 1 to 9, wherein a maximum carbon dioxide release temperature of the cyclic amine compound (A) is 140°C or less as measured by the following method:
heating the cyclic amine compound (A) with carbon dioxide absorbed from 23°C to 250°C at 10°C/minute of a heating rate, measuring a temperature at which an endothermic amount associated with desorption of the carbon dioxide reaches a maximum; and taking the temperature as the maximum carbon dioxide release temperature.

11. The carbon dioxide absorbent according to any one of claims 1 to 10, wherein a molecular weight of the cyclic amine compound (A) is 90 or more and 1,000 or less.

12. The carbon dioxide absorbent according to any one of claims 1 to 11, wherein an amine value of the cyclic amine compound (A) is 400 mgKOH/g or more and 1,500 mgKOH/g or less.

13. The carbon dioxide absorbent according to any one of claims 1 to 12, wherein the number of amino groups in the cyclic amine compound (A) is 1 or more and 6 or less.

14. The carbon dioxide absorbent according to any one of claims 1 to 13, wherein a cyclic structure of the cyclic amine compound (A) comprises at least one selected from the group consisting of a 5-membered ring and a 6-membered ring.

15. The carbon dioxide absorbent according to any one of claims 1 to 14, wherein the cyclic amine compound (A) comprises at least one selected from the group consisting of bis(aminomethyl)cyclohexane and derivatives thereof, limonenediamine and derivatives thereof, isophoronediamine and derivatives thereof, 2,5-bisaminomethylfuran and derivatives thereof, 2,5-bis(aminomethyl)tetrahydrofuran and derivatives thereof, furfurylamine and derivatives thereof, tetrahydrofurfurylamine and derivatives thereof, 4-aminomethyltetrahydropyran and derivatives thereof, 4-(2-aminoethyl)morpholine and derivatives thereof, and 2-thiophenemethylamine and derivatives thereof.

16. A method for capturing carbon dioxide, comprising using the carbon dioxide absorbent according to any one of claims 1 to 15.

17. The method according to claim 16, wherein the method comprises:
an absorption step of bringing a gas containing carbon dioxide into contact with the carbon dioxide absorbent, to cause the carbon dioxide absorbent to absorb the carbon dioxide, and
a desorption step of desorbing carbon dioxide from the carbon dioxide absorbent with carbon dioxide absorbed in the absorption step, and
wherein the desorption step comprises at least one step selected from the group consisting of the following (I) to (III):
(I) a step of subjecting the carbon dioxide absorbent with carbon dioxide absorbed to a reduced pressure condition;
(II) a step of bringing an inert gas not containing carbon dioxide into contact with the carbon dioxide absorbent with carbon dioxide absorbed; and
(III) a step of heating the carbon dioxide absorbent with carbon dioxide absorbed.

18. The method according to claim 17, wherein a temperature at which the gas containing carbon dioxide is brought into contact with the carbon dioxide absorbent in the absorption step is 0°C or more and less than 60°C.

19. The method according to claim 18, wherein a heating temperature in the step (III) is 50°C or more and 120°C or less.

20. A carbon dioxide separation and capture apparatus comprising:
an absorption apparatus comprising a mechanism for bringing a gas containing carbon dioxide into contact with the carbon dioxide absorbent according to any one of claims 1 to 15, to cause the carbon dioxide absorbent to absorb the carbon dioxide; and
a desorption apparatus comprising a mechanism for desorbing carbon dioxide from the carbon dioxide absorbent with carbon dioxide absorbed.
